(19)

Europäisches Patentamt

European Patent Office

Office européen des brevets

(11) **EP 3 895 676 A1**

(12) **EUROPEAN PATENT APPLICATION**
published in accordance with Art. 153(4) EPC

(43) Date of publication:
**20.10.2021 Bulletin 2021/42**

(21) Application number: **19895636.9**

(22) Date of filing: **12.12.2019**

(51) Int Cl.:
**A61F 13/53** *(2006.01)*     **C08F 20/06** *(2006.01)*
**C08J 3/12** *(2006.01)*

(86) International application number:
**PCT/JP2019/048795**

(87) International publication number:
**WO 2020/122202 (18.06.2020 Gazette 2020/25)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: 12.12.2018   JP 2018232724
            12.12.2018   JP 2018232843
            12.12.2018   JP 2018232847
            12.12.2018   JP 2018232726
            12.12.2018   JP 2018232728
            12.12.2018   JP 2018232848
            12.12.2018   JP 2018232850
            12.12.2018   JP 2018232851
            12.12.2018   JP 2018232856
            12.12.2018   JP 2018232857
            30.01.2019   JP 2019014525
            22.03.2019   JP 2019055283

(71) Applicant: **Sumitomo Seika Chemicals Co., Ltd.
Kako-gun, Hyogo 675-0145 (JP)**

(72) Inventor: **ITO Takashi
Himeji-shi, Hyogo 672-8076 (JP)**

(74) Representative: **Nederlandsch Octrooibureau
P.O. Box 29720
2502 LS The Hague (NL)**

(54) **ABSORBENT ARTICLE**

(57)     Disclosed is an absorbent article including: a laminate that has an absorbent containing water-absorbent resin particles and a fibrous material, and a core wrap sandwiching the absorbent from upper and lower sides of the absorbent; a liquid-permeable sheet disposed on an upper surface of the laminate; and a liquid-impermeable sheet disposed on a surface on a side opposite to the liquid-permeable sheet of the laminate, wherein the water-absorbent resin particles contain a crosslinked polymer having a monomer unit derived from an ethylenically unsaturated monomer including at least one compound selected from the group consisting of (meth)acrylic acid and a salt thereof, and inorganic particles. A proportion of (meth)acrylic acid and a salt thereof is 70 to 100 mol% with respect to a total amount of monomer units in the crosslinked polymer. The water-absorbent resin particles satisfy the following requirements (a) and (b). (a) A surface drying index of the absorbent article represented by the following formula is 45 or lower.

$$\text{Surface drying index} = (\text{moisture content index 1.5 minutes after liquid injection/moisture content index 1 minute after liquid injection}) \times 100$$

(b) A water retention capacity of the water-absorbent resin particles for a physiological saline solution is 30 g/g or more.

**Description**

**Technical Field**

The present invention relates to an absorbent article.

**Background Art**

Conventionally, an absorbent containing water-absorbent resin particles has been used in an absorbent article for absorbing a liquid containing water as a main component such as urine. In general, it has been strongly desired to inhibit occurrence of liquid leakage from an absorbent article during its use as much as possible (for example, Patent Literature 1, Patent Literature 2, and Patent Literature 3).

**Citation List**

**Patent Literature**

[Patent Literature 1] JP 2007-69161 A
[Patent Literature 2] JP 2006-167480 A
[Patent Literature 3] JP 2013-63254 A

**Summary of Invention**

**Technical Problem**

An object of the present invention is to provide an absorbent article in which occurrence of liquid leakage is reduced.

**Solution to Problem**

An absorbent article of the present invention includes an absorbent containing water-absorbent resin particles, in which the water-absorbent resin particles satisfy the following requirements (a) and (b).

(a) A surface drying index represented by the following formula is 45 or lower.

$$\text{Surface drying index} = (\text{moisture content index 1.5 minutes}$$
$$\text{after liquid injection/moisture content index 1 minute after liquid}$$
$$\text{injection}) \times 100$$

A moisture content index measurement method: 13.3 g of the water-absorbent resin particles and 12.9 g of pulverized pulp are uniformly mixed by air papermaking using an air flow type mixer (Padformer manufactured by O-TEC Co., Ltd.), and thereby a sheet-shaped absorbent having a size of 40 cm × 12 cm is obtained. Then, in a state where the absorbent is sandwiched from its upper and lower sides with two sheets of tissue paper, as core wraps, having a basis weight of 16 $g/m^2$ and having the same size as that of the absorbent, a load of 588 kPa is applied to the entire absorbent for 30 seconds to press it. Thereby, a laminate is obtained. An air-through type porous liquid-permeable sheet, which is made of polyethylene-polypropylene, has a basis weight of 22 $g/m^2$, and has the same size as that of the absorbent, is disposed on the upper surface of the laminate. A liquid-impermeable sheet, which is made of polyethylene and has the same size as that of the absorbent, is bonded to a surface on the side opposite to the air-through type porous liquid-permeable sheet, and thereby an absorbent article is obtained. 160 mL of a 0.9% by mass physiological saline solution (25°C) is injected into a center of the absorbent article using a liquid injection cylinder having an opening with an inner diameter of 3 cm, where a timing of the injection is defined as 0 minutes. A measurement probe (49 $mm^2$) is pressed on a center portion of the liquid injected site on the absorbent article, and a moisture content index at a surface of the center portion on the absorbent article is measured after a lapse of a predetermined time from the injection by an electrostatic capacitance method.
(b) A water retention capacity for a physiological saline solution is 30 g/g or more.

Regarding the absorbent article, a moisture content index of the water-absorbent resin particles 3 minutes after the liquid injection is preferably 0.
Regarding the absorbent article, a moisture content index of the water-absorbent resin particles 2 minutes after the liquid injection is preferably 2 or higher.
Regarding the absorbent article, a moisture content index of the water-absorbent resin particles 1 minute after the liquid injection is preferably 20 or higher.
The present invention further provides an absorbent article including: a laminate that has an absorbent containing water-absorbent resin particles and a fibrous material, and a core wrap sandwiching the absorbent from upper and lower sides of the absorbent; a liquid-permeable sheet that is disposed on an upper surface of the laminate; and a liquid-impermeable sheet that is disposed on a surface on a side opposite to the liquid-permeable sheet of the laminate. In the absorbent article, the water-absorbent resin particles contain a crosslinked polymer having a monomer unit derived from an ethylenically unsaturated monomer including at least one compound selected from the group consisting of (meth)acrylic acid and a salt thereof, and inorganic particles; a proportion of (meth)acrylic acid and a salt thereof is 70 to 100 mol% with respect to a total amount of monomer units in the crosslinked polymer; and the water-absorbent resin particles satisfy the following requirements (a) and (b).

(a) A surface drying index of the absorbent article represented by the following formula is 45 or lower.

$$\text{Surface drying index} = (\text{moisture content index 1.5 minutes after liquid injection/moisture content index 1 minute after liquid injection}) \times 100$$

A moisture content index measurement method: 160 mL of a 0.9% by mass physiological saline solution (25°C) is injected into a center of the absorbent article using a liquid injection cylinder having an opening with an inner diameter of 3 cm, where a timing of the injection is defined as 0 minutes. A measurement probe (49 mm$^2$) is pressed on a center portion of the liquid injected site on the absorbent article, and a moisture content index at a surface of the center portion on the absorbent article is measured after a lapse of a predetermined time from the injection by an electrostatic capacitance method.
(b) A water retention capacity of the water-absorbent resin particles for a physiological saline solution is 30 g/g or more.

The absorbent article preferably has a moisture content index 3 minutes after the liquid injection of 0.
The absorbent article preferably has a moisture content index 2 minutes after the liquid injection of 2 or higher.
The absorbent article preferably has a moisture content index 1 minute after the liquid injection of 20 or higher.

**Advantageous Effects of Invention**

According to the present invention, an absorbent article in which occurrence of liquid leakage is reduced is provided.

**Brief Description of Drawings**

Fig. 1 is a cross-sectional view showing an example of an absorbent article.
Fig. 2 is a schematic view showing a method for evaluating liquid leakage properties of an absorbent article.
Fig. 3 is a graph showing a moisture content index of an absorbent article in Examples.

**Description of Embodiments**

Hereinafter, suitable embodiments of the present invention will be described in detail. However, the present invention is not limited to the following embodiments.
In the present specification, "acrylic" and "methacrylic" are collectively referred to as "(meth)acrylic." "Acrylate" and "methacrylate" are also referred to as "(meth)acrylate." Regarding numerical value ranges described in a stepwise manner in the present specification, an upper limit value or a lower limit value of a numerical value range in a certain step can be arbitrarily combined with an upper limit value or a lower limit value of a numerical value range in another step. In a numerical value range described in the present specification, an upper limit value or a lower limit value of the numerical value range may be replaced with a value shown in examples. The term "water-soluble" means that a solubility of 5% by mass or more is exhibited in water at 25°C. For materials exemplified in the present specification, one kind may be used alone, or two or more kinds may be used in combination. In a case where there are a plurality of substances corresponding to each of components in a composition, a content of each of the components in the composition means a total amount of the plurality of substances present in the composition unless otherwise specified.

An absorbent article according to the present embodiment includes an absorbent containing water-absorbent resin particles. The water-absorbent resin particles satisfy the following requirement (a).

(a) A surface drying index represented by the following formula is 45 or lower.

$$\text{Surface drying index} = (\text{moisture content index 1.5 minutes after liquid injection/moisture content index 1 minute after liquid injection}) \times 100$$

In the present specification, the moisture content index is an index indicating a moisture content measured by an electrostatic capacitance method, and is indicated within a range of 0 to 120. The moisture content index can be measured using, for example, a comeometer. In the electrostatic capacitance method, by utilizing a significant difference present between permittivity of water and permittivity of another substance, a moisture content in the vicinity of a surface of the substance can be indicated as an index.

A moisture content index of the water-absorbent resin particles is measured by the following method. 13.3 g of the water-absorbent resin particles and 12.9 g of pulverized pulp are uniformly mixed by air papermaking using an air flow type mixer (Padformer manufactured by O-TEC Co., Ltd.), and thereby a sheet-shaped absorbent having a size of 40 cm × 12 cm is obtained. Then, in a state where the absorbent is sandwiched from its upper and lower sides with two sheets of tissue paper, as core wraps, having a basis weight of 16 g/m$^2$ and having the same size as that of the absorbent, a load of 588 kPa is applied to the entire absorbent for 30 seconds to press it. Thereby, a laminate is obtained. Furthermore, an air-through type porous liquid-permeable sheet, which is made of polyethylene-polypropylene, has a basis weight of 22 g/m$^2$, and has the same size as that of the absorbent, is disposed on the upper surface of the laminate. A liquid-impermeable sheet, which is made of polyethylene and has the same size as that of the absorbent, is bonded to a surface on the side opposite to the air-through type porous liquid-permeable sheet, and thereby an absorbent article is obtained. 160 mL of a 0.9% by mass physiological saline solution (25°C) is injected into a center of the absorbent article using a liquid injection cylinder having an opening with an inner diameter of 3 cm, where a timing of the injection is defined as 0 minutes. A measurement probe (49 mm$^2$) is pressed on a center portion of the liquid injected site on the absorbent article, and a moisture content index at a surface of the center portion on the absorbent article is measured after a lapse of a predetermined time from the injection by an electrostatic capacitance method.

Another aspect of the present invention relates to an absorbent article including a laminate that has an absorbent containing water-absorbent resin particles and a fibrous material, and a core wrap sandwiching the absorbent from upper and lower sides of the absorbent; a liquid-permeable sheet that is disposed on an upper surface of the laminate; and a liquid-impermeable sheet that is disposed on a surface on a side opposite to the liquid-permeable sheet of the laminate. A surface drying index of the absorbent article is 45 or lower. The surface drying index of the absorbent article is measured in the same manner as a surface drying index of the water-absorbent resin particles except that the absorbent article is used as it is as a measurement target of the surface drying index.

A surface drying index of the water-absorbent resin particles or absorbent article according to the present embodiment may be 10 or higher, 12 or higher, 15 or higher, 20 or higher, 30 or higher, or 35 or higher. A surface drying index thereof may be 42 or lower.

The water-absorbent resin particles or absorbent article according to the present embodiment preferably has a moisture content index 3 minutes after liquid injection which is an index measured by the above-described method of 0, from the viewpoint of further reducing occurrence of liquid leakage.

The water-absorbent resin particles or absorbent article according to the present embodiment preferably has a moisture content index 2 minutes after liquid injection which is an index measured by the above-described method of 2 or higher and more preferably 4 or higher. In a case where a moisture content index 2 minutes after liquid injection is within the above range, occurrence of liquid leakage is likely to be further reduced. A moisture content index 2 minutes after liquid injection may be 0 or higher.

Regarding the water-absorbent resin particles or absorbent article according to the present embodiment, a moisture content index 1.5 minutes after liquid injection which is an index measured by the above-described method may be, for example, 2 or higher, 4 or higher, 6 or higher, 10 or higher, 15 or higher, or 20 or higher. A moisture content index 1.5 minutes after liquid injection may be, for example, 60 or lower, 50 or lower, 40 or lower, 30 or lower, 20 or lower, or 15 or lower.

In the water-absorbent resin particles or absorbent article according to the present embodiment, a moisture content index 1 minute after liquid injection which is an index measured by the above-described method may be, for example, 20 or higher, 30 or higher, or 40 or higher. A moisture content index 1 minute after liquid injection may be, for example, 120 or lower, 100 or lower, 80 or lower, or 70 or lower.

The water-absorbent resin particles according to the present embodiment satisfy the following requirement (b). (b) A water retention capacity for a physiological saline solution is 30 g/g or more.

A water retention capacity of the water-absorbent resin particles for a physiological saline solution may be, for example, 30 g/g or more, 31 g/g or more, 32 g/g or more, 35 g/g or more, 37 g/g or more, 38 g/g or more, 39 g/g or more, or 40 g/g or more, from the viewpoint of appropriately increasing an absorption capacity of an absorbent. A water retention capacity of the water-absorbent resin particles for a physiological saline solution may be 60 g/g or less, 57 g/g or less, 55 g/g or less, 52 g/g or less, 50 g/g or less, 47 g/g or less, 45 g/g or less, 43 g/g, or 42 g/g or less. A water retention capacity for a physiological saline solution may be 30 to 60 g/g, 30 to 55 g/g, 30 to 50 g/g, 30 to 45 g/g, or 32 to 42 g/g. Furthermore, a water retention capacity for a physiological saline solution may be 31 to 60 g/g, 32 to 60 g/g, 35 to 60 g/g, 37 to 60 g/g, 39 to 55 g/g, 40 to 55 g/g, 40 to 52 g/g, or 40 to 50 g/g. The water retention capacity for a physiological saline solution is measured by a method described in Examples to be described later.

Examples of shapes of the water-absorbent resin particles include a substantially spherical shape, a crushed shape, and a granular shape. A median particle size of the water-absorbent resin particles may be 250 to 850 $\mu$m, 300 to 700 $\mu$m, or 300 to 600 $\mu$m. The water-absorbent resin particles according to the present embodiment may have a desired particle size distribution at a timing at which polymer particles are obtained by a production method to be described later, but their particle size distribution may be adjusted by performing operations such as adjustment of a particle size through classification with a sieve.

Regarding the type of the water-absorbent resin particles, commercially available products can be used. Examples thereof include a hydrolysate of a starch-acrylonitrile graft copolymer, a neutralized product of a starch-acrylic acid graft polymer, a saponified product of a vinyl acetate-acrylic acid ester copolymer, and a partially neutralized polyacrylic acid. Among them, a partially neutralized polyacrylic acid is preferable from the viewpoint of a production amount, manufacturing cost, a water absorption performance, and the like. Examples of methods of synthesizing the partially neutralized polyacrylic acid include a reverse-phase suspension polymerization method, an aqueous solution polymerization method, and the like. A degree of neutralization of the partially neutralized polyacrylic acid is preferably 50 mol% or more and is more preferably 70 to 90 mol% from the viewpoint of increasing an osmotic pressure of the water-absorbent resin particles and enhancing their water absorption capacity.

The water-absorbent resin particles according to the present embodiment can contain, for example, a crosslinked polymer formed by polymerization of monomers including ethylenically unsaturated monomers. The crosslinked polymer has a monomer unit derived from an ethylenically unsaturated monomer. That is, the water-absorbent resin particles according to the present embodiment may have a structural unit derived from ethylenically unsaturated monomers.

Examples of methods for polymerizing the monomers include a reverse-phase suspension polymerization method, an aqueous solution polymerization method, a bulk polymerization method, and a precipitation polymerization method. Among them, the reverse-phase suspension polymerization method or the aqueous solution polymerization method is preferable from the viewpoints of facilitating securement of favorable water absorption characteristics of the obtained water-absorbent resin particles and control of a polymerization reaction. Hereinbelow, a method for polymerizing ethylenically unsaturated monomers will be described with the reverse-phase suspension polymerization method as an example.

An ethylenically unsaturated monomer is preferably water-soluble. Examples thereof include (meth)acrylic acid and a salt thereof, 2-(meth)acrylamide-2-methylpropanesulfonic acid and a salt thereof, (meth)acrylamide, N,N-dimethyl (meth)acrylamide, 2-hydroxyethyl (meth)acrylate, N-methylol (meth)acrylamide, polyethylene glycol mono(meth)acrylate, N,N-diethylaminoethyl (meth)acrylate, N,N-diethylaminopropyl (meth)acrylate, and diethylaminopropyl (meth)acrylamide. In a case where an ethylenically unsaturated monomer has an amino group, the amino group may be quatemarized. A functional group such as a carboxyl group and an amino group, which is contained in the monomer, can function as a crosslinkable functional group in a surface crosslinking process to be described later. These ethylenically unsaturated monomers may be used alone or in a combination of two or more kinds thereof.

Among them, from the viewpoint of high industrial availability, the ethylenically unsaturated monomer preferably includes at least one compound selected from the group consisting of (meth)acrylic acid and a salt thereof, acrylamide, methacrylamide, and N,N-dimethyl acrylamide, and more preferably includes at least one compound selected from the group consisting of (meth)acrylic acid and a salt thereof, and acrylamide. The ethylenically unsaturated monomer more preferably includes at least one compound selected from the group consisting of (meth)acrylic acid and a salt thereof from the viewpoint of further enhancing water absorption characteristics.

For the monomer, a monomer other than the above-mentioned ethylenically unsaturated monomers may be partially used. Such a monomer can be used by, for example, being mixed with an aqueous solution containing the ethylenically unsaturated monomers. A usage amount of the ethylenically unsaturated monomers may be, for example, 70 to 100 mol%, may be 80 to 100 mol%, may be 90 to 100 mol%, may be 95 to 100 mol%, or may be 100 mol%, with respect to a total amount of monomers. Among them, a proportion of (meth)acrylic acid and a salt thereof may be 70 to 100 mol%, may be 80 to 100 mol%, may be 90 to 100 mol%, may be 95 to 100 mol%, or may be 100 mol%, with respect to a total amount of monomers.

Usually, the ethylenically unsaturated monomers are suitably used in a form of an aqueous solution. In general, it is sufficient for a concentration of the ethylenically unsaturated monomers in an aqueous solution containing the ethylenically unsaturated monomers (hereinafter, referred to as an aqueous solution of monomers) to be 20% by mass or more and a saturated concentration or less, and it is preferably 25% to 70% by mass, and is more preferably 30% to 55% by mass. Examples of water to be used include tap water, distilled water, and ion exchange water.

In a case where ethylenically unsaturated monomers to be used have an acidic group, an aqueous solution of monomers may be used after neutralizing this acidic group with an alkaline neutralizing agent. From the viewpoint of increasing an osmotic pressure of the obtained water-absorbent resin particles and thereby further enhancing water absorption characteristics such as a water retention capacity, a degree of neutralization in the ethylenically unsaturated monomers by the alkaline neutralizing agent is 10 to 100 mol%, is preferably 50 to 90 mol%, and is more preferably 60 to 80 mol% of the acidic group in the ethylenically unsaturated monomers. Examples of alkaline neutralizing agents include alkali metal salts such as sodium hydroxide, sodium carbonate, sodium hydrogen carbonate, potassium hydroxide, and potassium carbonate; and ammonia. These alkaline neutralizing agents may be used in a form of an aqueous solution to simplify a neutralizing operation. The above-mentioned alkaline neutralizing agents may be used alone or in combination of two or more kinds thereof. Neutralization of the acidic groups in the ethylenically unsaturated monomers can be performed by, for example, adding an aqueous solution of sodium hydroxide, potassium hydroxide, or the like dropwise to the aqueous solution of monomers and mixing them.

In the reverse-phase suspension polymerization method, an aqueous solution of monomers is dispersed in a hydrocarbon dispersion medium in the presence of a surfactant, and polymerization of ethylenically unsaturated monomers is performed using a radical polymerization initiator or the like.

Examples of surfactants include nonionic surfactants and anionic surfactants. Examples of nonionic surfactants include sorbitan fatty acid esters, (poly)glycerin fatty acid esters (where "(poly)" means both of a case with the prefix "poly" and a case without the prefix "poly," and the same applies hereinbelow), sucrose fatty acid esters, polyoxyethylene sorbitan fatty acid esters, polyoxyethylene glycerin fatty acid esters, sorbitol fatty acid esters, polyoxyethylene sorbitol fatty acid esters, polyoxyethylene alkyl ethers, polyoxyethylene alkylphenyl ethers, polyoxyethylene castor oil, polyoxyethylene hydrogenated castor oil, alkylallyl formaldehyde condensed polyoxyethylene ethers, polyoxyethylene polyoxypropylene block copolymers, polyoxyethylene polyoxypropyl alkyl ethers, and polyethylene glycol fatty acid esters. Examples of anionic surfactants include fatty acid salts, alkylbenzene sulfonate, alkylmethyl taurate, polyoxyethylene alkylphenyl ether sulfuric acid ester salts, polyoxyethylene alkyl ether sulfonic acid salts, phosphoric acid esters of polyoxyethylene alkyl ethers, and phosphoric acid esters of polyoxyethylene alkyl allyl ethers. Among them, the surfactant preferably includes at least one compound selected from the group consisting of sorbitan fatty acid esters, polyglycerin fatty acid esters, and sucrose fatty acid esters, from the viewpoints that a state of a W/O type reverse-phase suspension becomes favorable, water-absorbent resin particles are likely to be obtained with suitable particle sizes, and industrial availability becomes high. Furthermore, the surfactant more preferably includes sucrose fatty acid esters from the viewpoint that water absorption characteristics of the obtained water-absorbent resin particles are then improved. These surfactants may be used alone or in combination of two or more kinds thereof.

An amount of the surfactant is preferably 0.05 to 10 parts by mass, is more preferably 0.08 to 5 parts by mass, and is even more preferably 0.1 to 3 parts by mass, with respect to 100 parts by mass of the aqueous solution of the ethylenically unsaturated monomers, from the viewpoint that a sufficient effect is obtained within these usage amounts, and these amounts are economic.

Furthermore, a polymeric dispersant may be used in combination with the above-mentioned surfactant. Examples of polymeric dispersants include maleic anhydride-modified polyethylene, maleic anhydride-modified polypropylene, a maleic anhydride-modified ethylene-propylene copolymer, a maleic anhydride-modified EPDM (ethylene propylene diene terpolymer), maleic anhydride-modified polybutadiene, a maleic anhydride-ethylene copolymer, a maleic anhydride-propylene copolymer, a maleic anhydride-ethylene-propylene copolymer, a maleic anhydride-butadiene copolymer, polyethylene, polypropylene, an ethylene-propylene copolymer, oxidized polyethylene, oxidized polypropylene, an oxidized ethylene-propylene copolymer, an ethylene-acrylic acid copolymer, ethyl cellulose, ethyl hydroxyethyl cellulose, and the like. Among these polymeric dispersants, particularly from the viewpoint of dispersion stability of monomers, it is preferable to use maleic anhydride-modified polyethylene, maleic anhydride-modified polypropylene, a maleic anhydride-modified ethylene-propylene copolymer, a maleic anhydride-ethylene copolymer, a maleic anhydride-propylene copolymer, a maleic anhydride-ethylene-propylene copolymer, polyethylene, polypropylene, an ethylene-propylene copolymer, oxidized polyethylene, oxidized polypropylene, and an oxidized ethylene-propylene copolymer. These polymeric dispersants may be used alone or in combination of two or more kinds thereof.

An amount of the polymeric dispersant is preferably 0.05 to 10 parts by mass, is more preferably 0.08 to 5 parts by mass, and is even more preferably 0.1 to 3 parts by mass, with respect to 100 parts by mass of the aqueous solution of the ethylenically unsaturated monomers, from the viewpoint that a sufficient effect is obtained within these usage amounts, and these amounts are economic.

A radical polymerization initiator is preferably water-soluble. Examples thereof include persulfates such as potassium persulfate, ammonium persulfate, and sodium persulfate; peroxides such as methyl ethyl ketone peroxide, methyl isobutyl ketone peroxide, di-t-butyl peroxide, t-butyl cumyl peroxide, t-butyl peroxyacetate, t-butyl peroxyisobutyrate, t-butyl peroxypivalate, and hydrogen peroxide; and azo compounds such as 2,2'-azobis(2-amidinopropane) dihydrochloride, 2,2'-azobis[2-(N-phenylamidino)propane] dihydrochloride, 2,2'-azobis[2-(N-allylamidino)propane] dihydrochloride, 2,2'-azobis[2-(2-imidazolin-2-yl)propane] dihydrochloride, 2,2'-azobis{2-[1-(2-hydroxyethyl)-2-imidazolin-2-yl]propane} dihydrochloride, 2,2'-azobis{2-methyl-N-[1,1-bis(hydroxymethyl)-2-hydroxyethyl]propionamide}, 2,2'-azobis[2-methyl-N-(2-hydroxyethyl)-propionamide], and 4,4'-azobis(4-cyanovaleric acid). Among them, potassium persulfate, ammonium persulfate, sodium persulfate, 2,2'-azobis(2-amidinopropane) dihydrochloride, 2,2'-azobis[2-(2-imidazolin-2-yl)propane] dihydrochloride, and 2,2'-azobis{2-[1-(2-hydroxyethyl)-2-imidazolin-2-yl]propane} dihydrochloride are preferred. These radical polymerization initiators may be used alone or in combination of two or more kinds thereof.

A usage amount of the radical polymerization initiator may be 0.00005 to 0.01 moles with respect to 1 mole of the ethylenically unsaturated monomers. A case in which a usage amount of the radical polymerization initiator is 0.00005 moles or more is efficient, because then a polymerization reaction is not required to be performed for a long period of time. In a case where a usage amount thereof is 0.01 moles or less, a rapid polymerization reaction is unlikely to occur.

The radical polymerization initiator can also be used as a redox polymerization initiator when it is used in combination with a reducing agent such as sodium sulfite, sodium hydrogen sulfite, ferrous sulfate, and L-ascorbic acid.

In a polymerization reaction, a chain transfer agent may be contained in an aqueous solution of the ethylenically unsaturated monomers used for the polymerization. Examples of chain transfer agents include hypophosphites, thiols, thiolic acids, secondary alcohols, and amines.

Furthermore, a thickener may be contained in the aqueous solution of the ethylenically unsaturated monomers used for the polymerization to control a particle size of the water-absorbent resin particles. As the thickener, it is possible to use, for example, hydroxyethyl cellulose, hydroxypropyl cellulose, methyl cellulose, carboxymethyl cellulose, polyethylene glycol, polyacrylamide, polyethyleneimine, dextrin, sodium alginate, polyvinyl alcohol, polyvinylpyrrolidone, polyethylene oxide, and the like. In a case where stirring speeds in the polymerization are the same, a median particle size of particles to be obtained is likely to become large as a viscosity of the aqueous solution of the ethylenically unsaturated monomers becomes high.

The hydrocarbon dispersion medium may include at least one compound selected from the group consisting of a chained aliphatic hydrocarbon having 6 to 8 carbon atoms and an alicyclic hydrocarbon having 6 to 8 carbon atoms. Examples of hydrocarbon dispersion media include chained aliphatic hydrocarbons such as n-hexane, n-heptane, 2-methylhexane, 3-methylhexane, 2,3-dimethylpentane, 3-ethylpentane, and n-octane; alicyclic hydrocarbons such as cyclohexane, methylcyclohexane, cyclopentane, methylcyclopentane, trans-1,2-dimethylcyclopentane, cis-1,3-dimethylcyclopentane, and trans-1,3-dimethylcyclopentane; and aromatic hydrocarbons such as benzene, toluene, and xylene. These hydrocarbon dispersion media may be used alone or in combination of two or more kinds thereof. For the hydrocarbon dispersion medium, n-heptane, cyclohexane, or both n-heptane and cyclohexane may be contained, from the viewpoints of high industrial availability and stable qualities. Furthermore, from the same viewpoints, as a mixture of the hydrocarbon dispersion media, for example, a commercially available Exxsol Heptane (manufactured by ExxonMobil Chemical: containing n-heptane and 75% to 85% of hydrocarbons of isomers thereof) may be used.

A usage amount of the hydrocarbon dispersion medium is preferably 30 to 1,000 parts by mass, is more preferably 40 to 500 parts by mass, and is even more preferably 50 to 300 parts by mass, with respect to 100 parts by mass of the aqueous solution of monomers, from the viewpoint that polymerization heat is then appropriately removed, and thereby a polymerization temperature is easily controlled. In a case where a usage amount of the hydrocarbon dispersion medium is 30 parts by mass or more, there is a tendency that it becomes easy to control a polymerization temperature. In a case where a usage amount of the hydrocarbon dispersion medium is 1,000 parts by mass or less, there is a tendency that productivity of polymerization is improved, which is economic.

In general, internal crosslinking may occur by self-crosslinking upon the polymerization, but internal crosslinking may be carried out by further using an internal crosslinking agent, and thereby water absorption characteristics of the water-absorbent resin particles may be controlled. Examples of internal crosslinking agents to be used include di- or tri(meth)acrylic acid esters of polyols such as ethylene glycol, propylene glycol, trimethylolpropane, glycerin, polyoxyethylene glycol, polyoxypropylene glycol, and polyglycerin; unsaturated polyesters obtained by reacting the above mentioned polyols with unsaturated acids such as maleic acid and fumaric acid; bis(meth)acrylamides such as N,N'-methylenebis(meth)acrylamide; di- or tri(meth)acrylic acid esters obtained by reacting a polyepoxide with (meth)acrylic acid; carbamyl di(meth)acrylate esters obtained by reacting a polyisocyanate such as tolylene diisocyanate and hexamethylene diisocyanate with hydroxy ethyl (meth)acrylate; compounds having two or more polymerizable unsaturated groups, such as allylated starch, allylated cellulose, diallyl phthalate, N, N', N''-triallyl isocyanurate, , and divinylbenzene; polyglycidyl compounds such as (poly)ethylene glycol diglycidyl ether, (poly)propylene glycol diglycidyl ether, (poly)glycerin diglycidyl ether, (poly)glycerin triglycidyl ether, (poly)propylene glycol polyglycidyl ether, and polyglycerol polyglycidyl ether; haloepoxy compounds such as epichlorohydrin, epibromohydrin, and $\alpha$-methyl epichlorohydrin; and compounds having two or more reactive functional groups, such as isocyanate compounds including, for example, 2,4-tolylene diisocyanate and hexamethylene diisocyanate. Among these internal crosslinking agents, it is preferable to use a polyglycidyl compound, it is more preferable to use a diglycidyl ether compound, and it is particularly preferable to use (poly)ethylene glycol diglycidyl ether, (poly)propylene glycol diglycidyl ether, and (poly)glycerin diglycidyl ether. These crosslinking agents may be used alone or in combination of two or more kinds thereof.

An amount of the internal crosslinking agent is preferably 0 to 0.03 moles, is more preferably 0.00001 to 0.01 moles, and is even more preferably 0.00002 to 0.005 moles, per 1 mole of the ethylenically unsaturated monomer, from the viewpoints of inhibiting water-soluble properties by appropriately crosslinking the obtained polymer, and exhibiting a sufficient water absorption capacity.

An aqueous phase containing components such as an ethylenically unsaturated monomer, a radical polymerization initiator, and if necessary, an internal crosslinking agent; and an oil phase containing components such as a hydrocarbon dispersion medium, a surfactant, and if necessary, and a polymeric dispersant can be mixed and heated under stirring to carry out reverse-phase suspension polymerization in a water-in-oil system.

When performing the reverse-phase suspension polymerization, an aqueous solution of monomers which contains ethylenically unsaturated monomers is dispersed in a hydrocarbon dispersion medium in the presence of a surfactant and if necessary, a polymeric dispersant. In this case, a timing of adding the surfactant or the polymeric dispersant before the start of the polymerization reaction may be either before or after the addition of the aqueous solution of monomers.

Among them, it is preferable to carry out the polymerization after dispersing the aqueous solution of monomers in the hydrocarbon dispersion medium in which the polymeric dispersant has been dispersed, and then further dispersing the surfactant in the hydrocarbon dispersion medium, from the viewpoint that an amount of the hydrocarbon dispersion medium remaining in the obtained water-absorbent resin can then be easily reduced.

Such reverse-phase suspension polymerization can be carried out in one stage or in multiple stages of two or more stages. Furthermore, it is preferably carried out in two or three stages from the viewpoint of increasing productivity.

In a case where reverse-phase suspension polymerization is carried out in multiple stages of two or more stages, it is sufficient for stages after a second stage of reverse-phase suspension polymerization to be carried out in the same manner as in a first stage of reverse-phase suspension polymerization by adding ethylenically unsaturated monomers to a reaction mixture obtained in the first stage of polymerization reaction and mixing them, after performing the first stage of reverse-phase suspension polymerization. In reverse-phase suspension polymerization in each stage after the second stage, it is preferable to carry out reverse-phase suspension polymerization by adding, in addition to ethylenically unsaturated monomers, the above-mentioned radical polymerization initiator and internal crosslinking agent within a range of molar ratios of the respective components to the ethylenically unsaturated monomers, based on an amount of ethylenically unsaturated monomers added during reverse-phase suspension polymerization in each stage after the second stage. If necessary, the internal crosslinking agent may be used in reverse-phase suspension polymerization in each stage after the second stage. In a case where the internal crosslinking agent is used, it is preferable to carry out reverse-phase suspension polymerization by adding the internal crosslinking agent within a range of molar ratios of the respective components to the ethylenically unsaturated monomers based on an amount of ethylenically unsaturated monomers provided in each stage.

A temperature for the polymerization reaction varies depending on radical polymerization initiators used, but it is preferably 20°C to 150°C, and is more preferably 40°C to 120°C, from the viewpoint that the polymerization is then promptly performed, which shortens a polymerization time, and thereby economic efficiency increases, and that polymerization heat is then easily removed, and thereby the reaction is smoothly performed. A reaction time is generally 0.5 to 4 hours. Completion of the polymerization reaction can be confirmed from, for example, stop of temperature rising in the reaction system. Accordingly, a polymer of ethylenically unsaturated monomers is generally obtained in a state of a hydrous gel.

After the polymerization, post-polymerization crosslinking may be carried out by adding a crosslinking agent to the obtained hydrous gel polymer and heating them. By performing the post-polymerization crosslinking, a degree of crosslinking of the hydrous gel polymer can be increased, and thereby water absorption characteristics can be more preferably improved.

Examples of crosslinking agents for performing the post-polymerization crosslinking include polyols such as ethylene glycol, propylene glycol, 1,4-butanediol, trimethylolpropane, glycerin, polyoxyethylene glycol, polyoxypropylene glycol, and polyglycerin; compounds having two or more epoxy groups, such as (poly)ethylene glycol diglycidyl ether, (poly)propylene glycol diglycidyl ether, and (poly)glycerin diglycidyl ether; haloepoxy compounds such as epichlorohydrin, epibromohydrin, and α-methyl epichlorohydrin; compounds having two or more isocyanate groups such as 2,4-tolylene diisocyanate and hexamethylene diisocyanate; oxazoline compounds such as 1,2-ethylenebisoxazoline; carbonate compounds such as ethylene carbonate; and hydroxyalkylamide compounds such as bis[N,N-di(β-hydroxyethyl)]adipamide. Among them, polyglycidyl compounds such as (poly)ethylene glycol diglycidyl ether, (poly)glycerin diglycidyl ether, (poly)glycerin triglycidyl ether, (poly)propylene glycol polyglycidyl ether, and polyglycerol polyglycidyl ether are preferable. These crosslinking agents may be used alone or in combination of two or more kinds thereof.

An amount of the crosslinking agent used for the post-polymerization crosslinking is preferably 0 to 0.03 moles, is more preferably 0 to 0.01 moles, and is even more preferably 0.00001 to 0.005 moles, per 1 mole of the ethylenically unsaturated monomer, from the viewpoint of exhibiting suitable water absorption characteristics by appropriately crosslinking the obtained hydrous gel polymer. In a case where an amount of the crosslinking agent added is within the above range, it is possible to easily obtain water-absorbent resin particles or an absorbent article having a suitable surface drying index.

It is sufficient for a timing for adding the post-polymerization crosslinking to be after polymerization of ethylenically unsaturated monomers used for the polymerization. In a case of multi-stage polymerization, the crosslinking agent is preferably added after the multi-stage polymerization. From the viewpoint of a water content (to be described later), it is preferable to add the crosslinking agent for the post-polymerization crosslinking within a region of [water content immediately after polymerization ± 3% by mass], in consideration of heat generation during and after polymerization, retention due to process delay, system opening when a crosslinking agent is added, and fluctuation in moisture content due to addition of water associated with addition of a crosslinking agent.

Subsequently, drying is performed to remove moisture from the obtained hydrous gel polymer. By drying, polymer particles containing the polymer of ethylenically unsaturated monomers are obtained. Examples of drying methods include a method (a) in which the hydrous gel polymer in a state of being dispersed in a hydrocarbon dispersion medium is subjected to azeotropic distillation by heating from the outside, and the hydrocarbon dispersion medium is refluxed to remove moisture; a method (b) in which the hydrous gel polymer is taken out by decantation and dried under reduced pressure; and a method (c) in which the hydrous gel polymer is separated by filtration with a filter and dried under reduced pressure. Among them, the method (a) is preferably used for its simplicity in a production process.

Control over a particle size of the water-absorbent resin particle can be performed, for example, by adjusting a rotational speed of a stirrer during the polymerization reaction or by adding a powdery inorganic flocculating agent to the system after the polymerization reaction or at an initial time of drying. A particle size of the obtained water-absorbent resin particle can be increased by adding the flocculating agent. Examples of powdery inorganic flocculating agents include silica, zeolite, bentonite, aluminum oxide, talc, titanium dioxide, kaolin, clay, and hydrotalcite. Among them, silica, aluminum oxide, talc, or kaolin is preferable from the viewpoint of a flocculation effect.

In the reverse-phase suspension polymerization, the following method is preferable as a method of adding the powdery inorganic flocculating agent: a method in which a powdery inorganic flocculating agent is dispersed in a hydrocarbon dispersion medium of the same kind as that used in the polymerization, or water in advance, and then the mixture is mixed into a hydrocarbon dispersion medium containing a hydrous gel polymer under stirring.

In the production of the water-absorbent resin particles according to the present embodiment, a surface portion of the hydrous gel polymer is preferably crosslinked (surface-crosslinked) using a crosslinking agent in the drying process or any of subsequent processes. The surface crosslinking is preferably performed at a timing when the hydrous gel polymer has a specific water content. A timing of the surface crosslinking is preferably a time point at which a water content of the hydrous gel polymer is 5% to 50% by mass, is more preferably a time point at which a water content thereof is 10% to 40% by mass, and is even more preferably a time point at which a water content thereof is 15% to 35% by mass.

A water content (% by mass) of the hydrous gel polymer is calculated by the following formula.

$$\text{Water content} = [\text{Ww}/(\text{Ww} + \text{Ws})] \times 100$$

Ww: An amount of water of a hydrous gel polymer obtained by adding an amount of water used, as desired, upon mixing a powdery inorganic flocculating agent, a surface crosslinking agent, and the like to an amount obtained by subtracting an amount of water extracted to the outside of the system by the drying process from an amount of water contained in an aqueous liquid before polymerization in the all polymerization processes.

Ws: A solid fraction calculated from an amount of materials introduced, such as ethylenically unsaturated monomers, a crosslinking agent, and an initiator, each of which constitutes the hydrous gel polymer.

Examples of surface crosslinking agents for performing surface crosslinking include compounds having two or more reactive functional groups. Examples thereof include polyols such as ethylene glycol, propylene glycol, 1,4-butanediol, trimethylolpropane, glycerin, polyoxyethylene glycol, polyoxypropylene glycol, and polyglycerin; polyglycidyl compounds such as (poly)ethylene glycol diglycidyl ether, (poly)glycerin diglycidyl ether, (poly)glycerin triglycidyl ether, trimethylolpropane triglycidyl ether, (poly)propylene glycol polyglycidyl ether, and (poly)glycerol polyglycidyl ether; haloepoxy compounds such as epichlorohydrin, epibromohydrin, and $\alpha$-methyl epichlorohydrin; isocyanate compounds such as 2,4-tolylene diisocyanate and hexamethylene diisocyanate; oxetane compounds such as 3-methyl-3-oxetane methanol, 3-ethyl-3-oxetane methanol, 3-butyl-3-oxetane methanol, 3-methyl-3-oxetane ethanol, 3-ethyl-3-oxetane ethanol, and 3-butyl-3-oxetane ethanol; oxazoline compounds such as 1,2-ethylenebisoxazoline; carbonate compounds such as ethylene carbonate; and hydroxyalkylamide compounds such as bis[N,N-di($\beta$-hydroxyethyl)]adipamide. Among them, polyglycidyl compounds such as (poly)ethylene glycol diglycidyl ether, (poly)glycerin diglycidyl ether, (poly)glycerin triglycidyl ether, (poly)propylene glycol polyglycidyl ether, and polyglycerol polyglycidyl ether are more preferable. These surface crosslinking agents may be used alone or in combination of two or more kinds thereof.

In general, an amount of the surface crosslinking agent is preferably 0.00001 to 0.02 moles, is more preferably 0.00005 to 0.01 moles, and is even more preferably 0.0001 to 0.005 moles in a molar ratio, with respect to 1 mole of the ethylenically unsaturated monomer used in the polymerization, from the viewpoint of exhibiting suitable water absorption characteristics by appropriately crosslinking the obtained hydrous gel polymer.

A usage amount of the surface crosslinking agent is preferably 0.00001 moles or more from the viewpoint of sufficiently increasing a crosslinking density in a surface portion of the polymer particles and thereby enhancing gel strength of the water-absorbent resin particles. Furthermore, a usage amount thereof is preferably 0.02 moles or less from the viewpoint of easily obtaining a surface drying index within a suitable range and increasing a water retention capacity of the water-absorbent resin particles.

It is possible to obtain polymer particles, which are a surface-crosslinked dried product, by distilling off water and the hydrocarbon dispersion medium by a known method after the surface crosslinking reaction.

A ratio of an amount of an external crosslinking agent to an amount of the internal crosslinking agent (hereinafter, also referred to as a "crosslinking proportion") of the polymer particles is preferably 6 or more, is preferably 8 or more, and is more preferably 10 or more, from the viewpoint of improving liquid permeability and setting a surface drying index within a suitable range. A crosslinking proportion may be, for example, 100 or less, 80 or less, 60 or less, 40 or less, 30 or less, 20 or less, or 15 or less. An amount of the internal crosslinking agent is a total amount (mmol) of internal crosslinking agents added once or multiple times. An amount of the external crosslinking agent is a total amount (mmol) of an amount of a post-polymerization crosslinking agent and an amount of a surface crosslinking agent.

The water-absorbent resin particles according to the present embodiment may be composed of only the polymer particles, but they can further contain, for example, various additional components selected from inorganic powders, surfactants, oxidizers, reducing agents, metal chelating agents (ethylenediaminetetraacetic acid and its salts, diethylenetriaminepentaacetic acid and its salts, for example, diethylenetriaminepentaacetic acid pentasodium, and the like), radical chain inhibitors, antioxidants, antibacterial agents, deodorants, gel stabilizers, flowablility improvers (lubricants), and the like. The additional components may be disposed inside the polymer particles, on a surface of the polymer particles, or both of the inside and on the surface thereof. As the additional component, flowablility improvers (lubricants) are preferable, and among them, inorganic particles are more preferable. Examples of inorganic particles include silica particles such as amorphous silica. For example, flowablility of the water-absorbent resin particles can be improved by adding 0.05 to 5 parts by mass of amorphous silica as inorganic particles with respect to 100 parts by mass of the polymer particles. It is preferable that the water-absorbent resin particles according to the present embodiment do not contain non-porous spherical silicon oxide.

The water-absorbent resin particles may contain a plurality of inorganic particles disposed on the surface of the polymer particles. The inorganic particles can be disposed on the surface of the polymer particles by, for example, mixing the polymer particles and the inorganic particles. These inorganic particles may be silica particles such as amorphous silica. In a case where the water-absorbent resin particles contain inorganic particles disposed on the surface of the polymer particles, a ratio of the inorganic particles to a mass of the polymer particles may be 0.2% by mass or more, 0.5% by mass or more, 1.0% by mass or more, or 1.5% by mass or more, and it may be 5.0% by mass or less or 3.5% by mass or less. The inorganic particles referred to herein generally have a minute size as compared with a size of the polymer particles. For example, an average particle size of the inorganic particles may be 0.1 to 50 $\mu$m, 0.5 to 30 $\mu$m, or 1 to 20 $\mu$m. The average particle size referred to herein can be a value measured by a dynamic light scattering method or a laser diffraction/scattering method. In a case where an amount of the inorganic particles added is within the above range, it is easy to obtain water-absorbent resin particles having favorable water absorption characteristics.

An absorbent article according to the present embodiment includes an absorbent containing the water-absorbent resin particles. A content of the water-absorbent resin particles in the absorbent is preferably 100 to 1,000 g per square meter (that is, 100 to 1,000 g/m$^2$), is more preferably 150 to 800 g/m$^2$, and is even more preferably 200 to 700 g/m$^2$ of

the absorbent, from the viewpoint that sufficient liquid absorption performances are then obtained when the absorbent is used for the absorbent article. A content thereof is preferably 100 g/m$^2$ or more from the viewpoint of exhibiting sufficient liquid absorption performances as the absorbent article, and thereby particularly reducing liquid leakage. A content thereof is preferably 1,000 g/m$^2$ or less from the viewpoint of inhibiting occurrence of a gel blocking phenomenon, and thereby exhibiting a diffusion performance of a liquid as the absorbent article and further improving a permeation speed of the liquid.

The absorbent may further include, for example, a fibrous material in addition to the water-absorbent resin particles. The absorbent may be, for example, a mixture containing the water-absorbent resin particles and the fibrous material. A mass proportion of the water-absorbent resin particles in the absorbent may be 2% by mass to 100% by mass, is preferably 10% by mass to 80% by mass, and is more preferably 20% by mass to 70% by mass, with respect to a total of the water-absorbent resin particles and the fibrous material. The configuration of the absorbent may be, for example, a form in which water-absorbent resin particles and the fibrous materials are uniformly mixed, a form in which water-absorbent resin particles are held between fibrous materials formed in a sheet shape or a layer shape, or another form.

A content of the water-absorbent resin particles in the absorbent is preferably 100 to 1,000 g, is more preferably 150 to 800 g, and is even more preferably 200 to 700 g, per 1 m$^2$ of the absorbent from the viewpoint of easily obtaining a sufficient water absorption performance. A content of the fibrous material in the absorbent is preferably 50 to 800 g, is more preferably 100 to 600 g, and is even more preferably 150 to 500 g, per 1 m$^2$ of the absorbent from the viewpoint of easily obtaining a sufficient water absorption performance.

Examples of fibrous materials include finely pulverized wood pulp; cotton; cotton linter; rayon; cellulose-based fibers such as cellulose acetate; and synthetic fibers such as polyamides, polyesters, and polyolefins. The fibrous material may be a mixture of the above-mentioned fibers.

Fibers may be adhered to each other by adding an adhesive binder to the fibrous material in order to enhance shape retention properties before or during use of the absorbent. Examples of adhesive binders include thermal bonding synthetic fibers, hot-melt adhesives, and adhesive emulsions.

Examples of thermal bonding synthetic fibers include full-melt binders such as polyethylene, polypropylene, and an ethylene-propylene copolymer; and partial-melt binders formed of polypropylene and polyethylene in a side-by-side or core-and-sheath configuration. In the above-mentioned partial-melt binders, only a polyethylene portion is thermal-bonded. Examples of hot-melt adhesives include a blend of a base polymer such as an ethylene-vinyl acetate copolymer, a styreneisoprene-styrene block copolymer, a styrene-butadiene-styrene block copolymer, a styrene-ethylene-butylene-styrene block copolymer, a styrene-ethylene-propylene-styrene block copolymer, and an amorphous polypropylene with a viscosity imparting agent, a plasticizer, an antioxidant, or the like.

Examples of adhesive emulsions include polymers of at least one or more monomers selected from the group consisting of methyl methacrylate, styrene, acrylonitrile, 2-ethylhexyl acrylate, butyl acrylate, butadiene, ethylene, and vinyl acetate. These adhesive binders may be used alone or in combination of two or more kinds thereof.

The absorbent according to the present embodiment may further contain additives such as inorganic powders (for example, amorphous silica), deodorants, pigments, dyes, antibacterial agents, fragrances, and pressure sensitive adhesives. These additives can impart various functions to the absorbent. In a case where the water-absorbent resin particles contain inorganic particles, the absorbent may contain an inorganic powder in addition to the inorganic particles in the water-absorbent resin particles. Examples of inorganic powders include silicon dioxide, zeolite, kaolin, clay, and the like.

A shape of the absorbent according to the present embodiment is not particularly limited, but it may be, for example, a sheet shape. A thickness of the absorbent (for example, a thickness of a sheet-shaped absorbent) may be, for example, 0.1 to 20 mm or 0.3 to 15 mm.

The absorbent article according to the present embodiment may include, for example, a core wrap, a liquid-permeable top sheet, and a liquid-impermeable back sheet, in addition to the absorbent. The core wrap retains the shape of the absorbent. The liquid-permeable top sheet is disposed on the outermost part on a side from which an absorption target liquid is infiltrated. The liquid-impermeable back sheet is disposed on the outermost part on a side opposite to the side from which the absorption target liquid is infiltrated.

Examples of the absorbent article include diapers (for example, paper diapers), toilet training pants, incontinence pads, sanitary products (sanitary napkins, tampons, and the like), sweat pads, pet sheets, portable toilet members, animal excrement treatment materials, and the like.

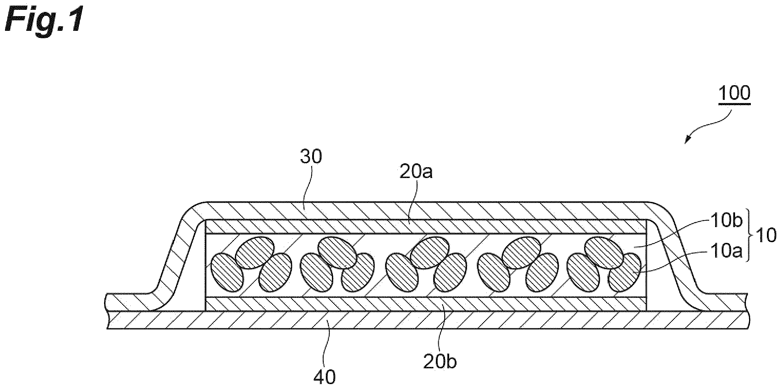

Fig. 1 is a cross-sectional view showing an example of an absorbent article. An absorbent article 100 shown in Fig. 1 includes an absorbent 10, core wraps 20a and 20b, a liquid-permeable top sheet 30, and a liquid-impermeable back sheet 40. In the absorbent article 100, the liquid-impermeable back sheet 40, the core wrap 20b, the absorbent 10, the core wrap 20a, and the liquid-permeable top sheet 30 are laminated in this order. In Fig. 1, there is a portion shown to be a gap between the members, but the members may be in close contact with each other without the gap.

The absorbent 10 has water-absorbent resin particles 10a and a fiber layer 10b containing a fibrous material.

The water-absorbent resin particles 10a are dispersed in the fiber layer 10b.

The core wrap 20a is disposed on one surface side of the absorbent 10 (an upper side of the absorbent 10 in Fig. 1) in a state of being in contact with the absorbent 10. The core wrap 20b is disposed on the other surface side of the absorbent 10 (a lower side of the absorbent 10 in Fig. 1) in a state of being in contact with the absorbent 10. The absorbent 10 is disposed between the core wrap 20a and the core wrap 20b.

The core wrap 20a and the core wrap 20b each have, for example, a main surface having the same size as that of the absorbent 10. By using the core wraps, it is possible to maintain shape retainability of the absorbent and prevent the water-absorbent resin particles and the like constituting the absorbent from falling off and flowing. Examples of the core wraps include non-woven fabrics, woven fabrics, tissues, synthetic resin films having liquid permeation holes, net shaped sheets having a mesh, and the like, of which tissues obtained by wet-type molding pulverized pulp are preferable from the viewpoint of economic efficiency.

The liquid-permeable top sheet 30 is disposed on the outermost part on a side from which an absorption target liquid is infiltrated. The liquid-permeable top sheet 30 is disposed on the core wrap 20a in a state of being in contact with the core wrap 20a. The liquid-impermeable back sheet 40 is disposed on the outermost part on a side opposite to the liquid-permeable top sheet 30, in the absorbent article 100. The liquid-impermeable back sheet 40 is disposed below the core wrap 20b in a state of being in contact with the core wrap 20b. The liquid-permeable top sheet 30 and the liquid-impermeable back sheet 40 each have, for example, a main surface wider than the main surface of the absorbent 10, and outer edges of the liquid-permeable top sheet 30 and the liquid-impermeable back sheet 40 respectively extend around the absorbent 10 and the core wraps 20a and 20b.

Examples of the liquid-permeable top sheet 30 include non-woven fabrics, porous sheets, and the like. Examples of non-woven fabrics include thermal bonded non-woven fabrics, air through non-woven fabrics, resin bonded non-woven fabrics, spunbond non-woven fabrics, melt-blown non-woven fabrics, spunbond/melt-blown/spunbond non-woven fabrics, airlaid non-woven fabrics, spunlace non-woven fabrics, point-bonded non-woven fabrics, and the like. Among them, thermal bonded non-woven fabrics, air through non-woven fabrics, spunbond non-woven fabrics, and spunbond/melt-blown/spunbond non-woven fabrics are preferably used.

As constituent materials for the liquid-permeable top sheet 30, it is possible to use resins or fibers known in the technical field. Examples thereof include polyolefins such as polyethylene (PE) and polypropylene (PP); polyesters such as polyethylene terephthalate (PET), polytrimethylene terephthalate (PTT), and polyethylene naphthalate (PEN); polyamides such as nylon; rayon; other synthetic resins or synthetic fibers; and fibers such as cotton, silk, hemp, and pulp (cellulose), from the viewpoint of liquid permeability, flexibility, and strength when the liquid-permeable top sheet 30 is used in an absorbent article. As the constituent material, synthetic fibers are preferably used from the viewpoint of increasing strength of the liquid-permeable top sheet 30. Among them, polyolefins and polyesters are preferable. These materials may be used alone or in combination of two or more materials.

It is desirable that a non-woven fabric used for the liquid-permeable top sheet 30 have appropriate hydrophilicity from the viewpoint of improving liquid absorption performances of the absorbent article. From this viewpoint, a non-woven fabric having a hydrophilicity of 5 to 200 is preferable, and a non-woven fabric having a hydrophilicity of 10 to 150 is more preferable, where the hydrophilicity is measured according to "Hydrophilicity of Non-woven fabric" (in accordance with Pulp and Paper Test Method No. 68 (2000)) disclosed in PCT International Publication No. WO2011/086843. Among the above-mentioned non-woven fabrics, a non-woven fabric having such a hydrophobicity may be formed of a material, such as rayon fibers, which shows an appropriate hydrophilicity by itself; or may be formed of fibers obtained by hydrophilizing hydrophobic chemical fibers such as polyolefin fibers and polyester fibers by a known method and imparting an appropriate hydrophilicity thereto.

Examples of methods of hydrophilizing chemical fibers include a method of obtaining a non-woven fabric by a spunbond technique from a mixture in which a hydrophilizing agent is added to hydrophobic chemical fibers in a spunbond non-woven fabric, a method of using a hydrophilizing agent when producing a spunbond non-woven fabric from hydrophobic chemical fibers, and a method of obtaining a spunbond non-woven fabric from hydrophobic chemical fibers, and thereafter impregnating the spunbond non-woven fabric with a hydrophilizing agent. As the hydrophilizing agent, the following examples are used: anionic surfactants such as aliphatic sulfonic acid salts and higher alcohol sulfuric acid ester salts; cationic surfactants such as quaternary ammonium salts; nonionic surfactants such as polyethylene glycol fatty acid esters, polyglycerin fatty acid esters, and sorbitan fatty acid esters; silicone surfactants such as polyoxyalkylene-modified silicone; stain release agents formed of polyester-based, polyamide-based, acrylic-based, or urethane-based resin; and the like.

It is preferable that a non-woven fabric used for the liquid-permeable top sheet 30 be moderately bulky and have a large weight per unit area from the viewpoint of imparting favorable liquid permeability, flexibility, strength, and cushioning properties to an absorbent article, and accelerating a liquid penetration speed of an absorbent article. A weight per unit area of the non-woven fabric is preferably 5 to 200 g/m$^2$, is more preferably 8 to 150 g/m$^2$, and is even more preferably 10 to 100 g/m$^2$. Furthermore, a thickness of the non-woven fabric is preferably 20 to 1,400 μm, is more preferably 50 to 1,200 μm, and is even more preferably 80 to 1,000 μm.

The liquid-impermeable back sheet 40 prevents a liquid absorbed by the absorbent 10 from leaking to the outside from the back sheet 40 side. For the liquid-impermeable back sheet 40, it is possible to use liquid-impermeable films mainly composed of polyolefin resins such as polyethylene (PE) and polypropylene (PP); breathable resin films; composite films in which a breathable resin film is bonded to a non-woven fabric such as spunbond non-woven fabric and spunlace non-woven fabric; spunbond/melt-blown/spunbond (SMS) non-woven fabrics in which a water-resistant melt blown non-woven fabric is sandwiched between high-strength spunbond non-woven fabrics; and the like. For the back sheet 40, it is possible to use a resin film having a weight per unit area of 10 to 50 g/m$^2$ and mainly made of low-density polyethylene (LDPE) resin from the viewpoint of ensuring flexibility so as not to impair a sensation of wearing the absorbent article. Furthermore, in a case where a breathable material is used, dampness generated when wearing the absorbent article is reduced, and thereby discomfort to a wearer can be reduced.

A magnitude relationship between the absorbent 10, the core wraps 20a and 20b, the liquid-permeable top sheet 30, and the liquid-impermeable back sheet 40 is not particularly limited, and it is appropriately adjusted according to usage applications and the like of the absorbent article. Furthermore, a method of retaining the shape of the absorbent 10 using the core wraps 20a and 20b is not particularly limited. The absorbent may be sandwiched by a plurality of the core wraps as shown in Fig. 1, or the absorbent may be covered with one core wrap.

The absorbent 10 may be adhered to the liquid-permeable top sheet 30. By adhering the absorbent 10 to the liquid-permeable top sheet 30, a liquid is more smoothly guided to the absorbent, and thereby it becomes easy to obtain an absorbent article that is further excellent in preventing liquid leakage. In a case where the absorbent 10 is sandwiched or covered by the core wrap, it is preferable that at least the core wrap and the liquid-permeable top sheet 30 be adhered to each other, and it is more preferable that the core wrap and the absorbent 10 be adhered to each other in addition to the adhesion of the core wrap and the liquid-permeable top sheet 30. Examples of methods of adhesion include a method of adhesion by applying a hot-melt adhesive to the liquid-permeable top sheet 30 in shapes such as a vertical stripe shape and a spiral shape at predetermined intervals in a width direction; a method of adhesion using a water-soluble binder selected from starch, carboxymethyl cellulose, polyvinyl alcohol, polyvinylpyrrolidone, and other water-soluble polymers; and the like. Furthermore, a method of adhesion by thermal bonding may be adopted in a case where the absorbent 10 contains thermal bonding synthetic fibers.

[Examples]

Hereinafter, the present invention will be described in more detail with reference to examples, but the present invention is not limited to these examples.

<Production of water-absorbent resin particles>

[Example 1] A cylindrical round-bottomed separable flask was prepared, which had an inner diameter of 11 cm and a capacity of 2 L, and was equipped with a reflux condenser, a dropping funnel, a nitrogen gas introduction tube, and as a stirrer, a stirring blade having four inclined paddle blades, each having a blade diameter of 5 cm, in a two-tier manner. 293 g of n-heptane as a hydrocarbon dispersion medium was weighed into this flask, and 0.736 g of a maleic anhydride-modified ethylene-propylene copolymer (HI-WAX 1105A, Mitsui Chemicals, Inc.) as a polymeric dispersant was added thereinto. The reaction solution in the flask was heated to 80°C while being stirred to dissolve the polymeric dispersant. Thereafter, the reaction solution was cooled to 50°C.

Meanwhile, 92.0 g (1.03 moles) of an aqueous solution of 80.5% by mass acrylic acid as an ethylenically unsaturated monomer was weighed into a beaker with an internal capacity of 300 mL, and 147.7 g of an aqueous solution of 20.9% by mass sodium hydroxide was added dropwise thereto while cooling the beaker from the outside to perform neutralization to 75 mol%. Thereafter, 0.092 g of hydroxylethyl cellulose (HEC AW-15F, manufactured by Sumitomo Seika Chemicals Co., Ltd.) as a thickener, 0.092 g (0.339 mmol) of 2,2'-azobis(2-amidinopropane) dihydrochloride and 0.018 g (0.068 mmol) of potassium persulfate as a radical polymerization initiator, and 0.010 g (0.057 mmol) of ethylene glycol diglycidyl ether as an internal crosslinking agent were added and dissolved to prepare a first-stage aqueous liquid.

The prepared aqueous liquid was added into the reaction solution in the separable flask and stirred for 10 minutes. Then, a surfactant solution, in which 0.736 g of sucrose stearic acid ester (HLB: 3, Mitsubishi-Chemical Foods Corporation, RYOTO Sugar Ester S-370) as a surfactant was dissolved in 6.62 g of n-heptane by heating, was further added into the reaction solution. While stirring the reaction solution at 550 rpm as a rotational speed of the stirrer, the inside of the system was sufficiently replaced with nitrogen. Thereafter, the flask was immersed in a water bath at 70°C to raise its temperature, and polymerization was performed for 60 minutes. Thereby, a first-stage polymerization slurry liquid was obtained.

Meanwhile, 128.8 g (1.43 moles) of an aqueous solution of 80.5% by mass acrylic acid as an ethylenically unsaturated monomer was weighed into another beaker with an internal capacity of 500 mL, and 159.0 g of an aqueous solution of 27% by mass sodium hydroxide was added dropwise thereto while cooling the beaker from the outside to perform neutralization to 75 mol%. Thereafter, as a radical polymerization initiator, 0.129 g (0.475 mmol) of 2,2'-azobis(2-amidinopropane) dihydrochloride and 0.026 g (0.095 mmol) of potassium persulfate were added and dissolved to prepare a second-stage aqueous liquid.

While stirring at 1,000 rpm as a rotational speed of the stirrer, the inside of the separable flask system was cooled to 25°C. Then, a total amount of the second-stage aqueous liquid was added into the first-stage polymerization slurry liquid in the separable flask, and the inside of the system was replaced with nitrogen for 30 minutes. Thereafter, the flask was immersed in a water bath at 70°C again to raise its temperature, and a polymerization reaction was performed for 60 minutes. Thereafter, 0.580 g (0.067 mmol) of an aqueous solution of 2% by mass ethylene glycol diglycidyl ether was added as a crosslinking agent for post-polymerization crosslinking, and thereby a hydrous gel polymer was obtained.

Under stirring, 0.265 g of an aqueous solution of 45% by mass diethylenetriaminepentaacetic acid pentasodium was added into the reaction solution containing the hydrous gel polymer obtained after the second-stage polymerization. Thereafter, the flask was immersed in an oil bath set to 125°C, and 238.5 g of water was extracted out of the system while n-heptane was refluxed by azeotropic distillation with n-heptane and water. Thereafter, 4.42 g (0.507 mmol) of an aqueous solution of 2% by mass ethylene glycol diglycidyl ether as a surface crosslinking agent was added into the flask, and maintained at 83°C for 2 hours.

Thereafter, n-heptane was evaporated at 125°C, and the residue was dried to obtain polymer particles (dried product). These polymer particles were passed through a sieve having an aperture of 850 $\mu$m, and 0.2% by mass of amorphous silica (Oriental Silicas Corporation, Tokusil NP-S) with respect to a mass of the polymer particles was mixed with the polymer particles. Thereby, 232.1 g of water-absorbent resin particles containing the amorphous silica was obtained. A median particle size of the water-absorbent resin particles was 396 $\mu$m. A ratio of the amount of the external crosslinking agent to the amount of the internal crosslinking agent (crosslinking proportion) was 10.1. An amount of the internal crosslinking agent is a total amount (mmol) of internal crosslinking agents added once or twice. An amount of the external crosslinking agent is a total amount (mmol) of an amount of a post-polymerization crosslinking agent and an amount of a surface crosslinking agent.

[Example 2] 236.3 g of water-absorbent resin particles were obtained in the same manner as in Example 1 except that a blending amount of amorphous silica with respect to polymer particles (dried product) was changed to 2.0% by mass. A median particle size of the water-absorbent resin particles was 393 $\mu$m. A crosslinking proportion was 10.1.

[Example 3] 231.0 g of water-absorbent resin particles was obtained in the same manner as in Example 1 except that, in preparation of a first-stage aqueous liquid, an amount of potassium persulfate as a radical polymerization initiator was changed to 0.0736 g (0.272 mmol), but 2,2'-azobis(2-amidinopropane) dihydrochloride was not used; in preparation of a second-stage aqueous liquid, an amount of potassium persulfate as a radical polymerization initiator was changed to 0.090 g (0.334 mmol), but 2,2'-azobis(2-amidinopropane) dihydrochloride was not used; in a reaction solution containing a hydrous gel polymer obtained after second-stage polymerization, an amount of water extracted out of the system by azeotropic distillation was changed to 247.9 g; and a blending amount of amorphous silica with respect to polymer particles was changed to 0.5% by mass. A median particle size of the water-absorbent resin particles was 355 $\mu$m. A crosslinking proportion was 10.1.

[Example 4] 229.2 g of water-absorbent resin particles was obtained in the same manner as in Example 1 except that, in preparation of a first-stage aqueous liquid, an amount of potassium persulfate as a radical polymerization initiator was changed to 0.0736 g (0.272 mmol), but 2,2'-azobis(2-amidinopropane) dihydrochloride was not used; in preparation of a second-stage aqueous liquid, an amount of potassium persulfate as a radical polymerization initiator was changed to 0.090 g (0.334 mmol), but 2,2'-azobis(2-amidinopropane) dihydrochloride was not used; in a reaction solution containing a hydrous gel polymer obtained after second-stage polymerization, an amount of water extracted out of the system by azeotropic distillation was changed to 239.7 g; and a blending amount of amorphous silica with respect to polymer particles was changed to 0.5% by mass. A median particle size of the water-absorbent resin particles was 377 $\mu$m. A crosslinking proportion was 10.1.

[Comparative Example 1]229.6 g of water-absorbent resin particles was obtained in the same procedure as in Example 1 except that, in preparation of a first-stage aqueous liquid, an amount of ethylene glycol diglycidyl ether as an internal crosslinking agent was changed to 0.0046 g (0.026 mmol); in preparation of a second-stage aqueous solution, 0.0116 g (0.067 mmol) of ethylene glycol diglycidyl ether was added as an internal crosslinking agent; a crosslinking agent for post-polymerization crosslinking was not added; and for a reaction solution containing a hydrous gel polymer obtained after second-stage polymerization, an amount of water extracted out of the system by azeotropic distillation was changed to 219.2 g, and an amount of an aqueous solution of 2% by mass ethylene glycol diglycidyl ether as a surface crosslinking agent was changed to 6.62 g (0.761 mmol). A median particle size of the water-absorbent resin particles was 356 $\mu$m. A crosslinking proportion was 8.2.

[Comparative Example 2]229.6 g of water-absorbent resin particles was obtained in the same manner as in Example 1 except that, in preparation of a first-stage aqueous liquid, an amount of ethylene glycol diglycidyl ether as an internal crosslinking agent was changed to 0.0046 g (0.026 mmol); in preparation of a second-stage aqueous liquid, 0.0116 g (0.067 mmol) of ethylene glycol diglycidyl ether was added as an internal crosslinking agent; a crosslinking agent for post-polymerization crosslinking was not added; and in a reaction solution containing a hydrous gel polymer obtained after second-stage polymerization, an amount of water extracted out of the system by azeotropic distillation was changed to 234.2 g. A median particle size of the water-absorbent resin particles was 355 $\mu$m. A crosslinking proportion was 5.5.

[Comparative Example 3]222.2 g of water-absorbent resin particles was obtained in the same manner as in Example 1 except that, in preparation of a first-stage aqueous liquid, an amount of ethylene glycol diglycidyl ether as an internal crosslinking agent was changed to 0.0368 g (0.211 mmol); in preparation of a second-stage aqueous liquid, an amount of ethylene glycol diglycidyl ether as an internal crosslinking agent was changed to 0.0515 g (0.296 mmol); a crosslinking agent for post-polymerization crosslinking was not added; and in a hydrous gel polymer obtained after second-stage polymerization, an amount of water extracted by azeotropic distillation was changed to 286.9 g. A median particle size of the particles was 396 $\mu$m. A crosslinking proportion was 1.0.

The obtained water-absorbent resin particles were evaluated for a water retention capacity for a physiological saline solution, a median particle size, a surface drying index, and gradient absorption by the following method.

<Measurement of water retention capacity for physiological saline solution> A cotton bag (cotton broadcloth No. 60, 100 mm in width × 200 mm in length) into which 2.0 g of the water-absorbent resin particles had been weighed was placed in a beaker having a capacity of 500 mL. 500 g of an aqueous solution of 0.9% by mass sodium chloride (physiological saline solution) was poured into the cotton bag containing the water-absorbent resin particles at once so that a lump could not be produced. The upper part of the cotton bag was bound with a rubber band and left to stand for 30 minutes, and thereby the water-absorbent resin particles were swollen. The cotton bag after an elapse of 30 minutes was dehydrated for 1 minute using a dehydrator (manufactured by KOKUSAN Co., Ltd., product number: H-122) which had been set at a centrifugal force of 167 G, and a mass Wa (g) of the dehydrated cotton bag containing the swollen gel was measured. By performing the same operation without addition of the water-absorbent resin particles, a mass Wb (g) of an empty cotton bag upon moisturizing was measured, and a water retention capacity for a physiological saline solution was calculated by the following formula. The results are shown in Table 1.

$$\text{Water retention capacity for physiological saline solution (g/g)}$$
$$= [\text{Wa} - \text{Wb}]/2.0$$

<Measurement of median particle size (particle size distribution)> 50 g of the water-absorbent resin particles was used for measuring a median particle size (particle size distribution). JIS standard sieves were combined in the following order from the top: a sieve having an aperture of 850 $\mu$m, a sieve having an aperture of 500 $\mu$m, a sieve having an aperture of 425 $\mu$m, a sieve having an aperture of 300 $\mu$m, a sieve having an aperture of 250 $\mu$m, a sieve having an aperture of 180 $\mu$m, a sieve having an aperture of 150 $\mu$m, and a receiving tray.

The water-absorbent resin particles were fed to the topmost sieve among the combination of the sieves, shaken for 20 minutes using a Ro-Tap shaker, and thereby classified. After the classification, a mass of the water-absorbent resin particles remaining on each of the sieves was calculated as a mass percentage with respect to a total amount to determine a particle size distribution. By integrating values on the sieves in descending order of the particle sizes with regard to the particle size distribution, a relationship between the aperture of the sieve and the integrated value of mass percentages of the water-absorbent resin particles remaining on the sieve was plotted on a log-probability paper. The plotted points on the probability paper were connected with straight lines, and a particle size corresponding to 50% by mass of the integrated mass percentage was taken as a median particle size.

<Measurement of surface drying index>

[Production of absorbent article] 13.3 g of the water-absorbent resin particles and 12.9 g of pulverized pulp were uniformly mixed by air papermaking using an air flow type mixer (Padformer manufactured by O-TEC Co., Ltd.), and thereby a sheet-shaped absorbent having a size of 40 cm × 12 cm was produced. Then, in a state where the absorbent was sandwiched from its upper and lower sides with two sheets of tissue paper, as core wraps, having a basis weight of 16 g/m$^2$ and having the same size as that of the absorbent, a load of 588 kPa was applied to the entire absorbent for 30 seconds to press it. Thereby, a laminate was obtained. An air-through type porous liquid-permeable sheet, which was made of polyethylene-polypropylene, had a basis weight of 22 g/m$^2$, and had the same size as that of the absorbent, was disposed on the upper surface of the laminate. Furthermore, a liquid-impermeable sheet, which was made of polyethylene and had the same size as that of the absorbent, was bonded to a surface on the side opposite to the air-through type porous liquid-permeable sheet, and thereby an absorbent article was produced.

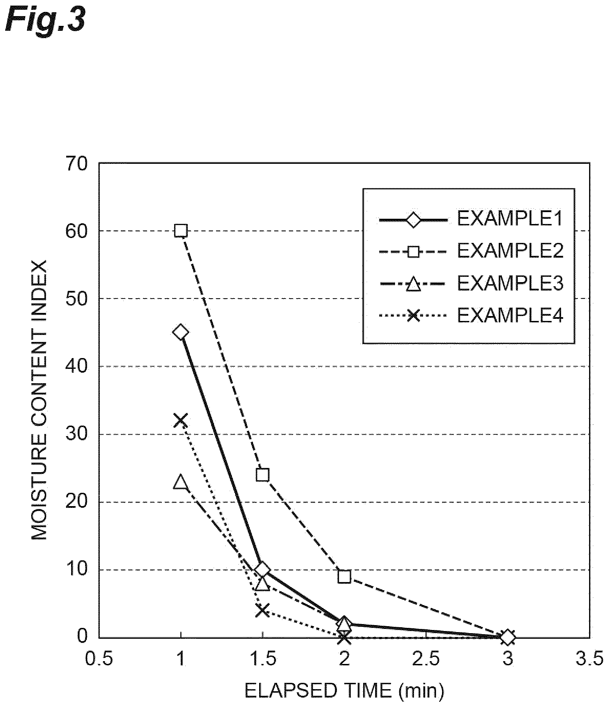

[Measurement of moisture content index] 160 mL of a 0.9% by mass physiological saline solution (25°C) was prepared in a 200 mL graduated cylinder, and injected into the center of the absorbent article for testing using a liquid injection cylinder having an opening with an inner diameter of 3 cm. A timing of the injection was defined as 0 minutes. A moisture content after a lapse of a predetermined time from the liquid injection was measured using a Corneometer (manufactured by Courage + Khazaka electronic GmbH, CM825). Specifically, a moisture content measurement probe (49 mm$^2$) attached to the Corneometer was pressed on a center portion of the liquid injected site on the absorbent article, and moisture content indexes on the surface of the absorbent article 1 minute, 1.5 minutes, 2 minutes, 3 minutes, and 4 minutes after the injection of the physiological saline solution were measured with a constant force (IN ± 10%). The measurement was performed in the environment of 25°C ± 1°C and a relative humidity of 50% ± 5%. The results are shown in Table 1 and Fig. 3.

From the obtained measurement values, a surface drying index was calculated by the following formula.

$$\text{Surface drying index} = (\text{moisture content index 1.5 minutes after liquid injection/moisture content index 1 minute after liquid injection}) \times 100$$

<Test for evaluating leakage properties>

(1) Preparation of artificial urine

Sodium chloride, calcium chloride, and magnesium sulfate, each at the following concentration, were dissolved in ion exchange water. A small amount of Blue No. 1 was added to the obtained solution to obtain artificial urine colored in blue. The obtained artificial urine was used as a test solution for evaluating leakage properties. The following concentrations are concentrations based on a total mass of the artificial urine.

Composition of artificial urine

NaCl: 0.780% by mass
$CaCl_2$: 0.022% by mass
$MgSO_4$: 0.038% by mass
Blue No. 1: 0.002% by mass

(2) Slope absorption test

10 g of the water-absorbent resin particles and 9.5 g of pulverized pulp were uniformly mixed by air papermaking, and thereby a sheet-shaped absorbent having a size of 12 cm × 32 cm was produced. The absorbent was placed on a core wrap (tissue paper) having a basis weight of 16 g/m$^2$, and a liquid-permeable top sheet and an upper part core wrap (tissue paper) were placed on the absorbent. As the liquid-permeable top sheet and the upper part core wrap, those collected from a hygiene product (MamyPoko Pants L size) which was commercially available in Japan were used. A load of 588 kPa was applied for 30 seconds to the absorbent sandwiched between the core wrap and the liquid-permeable top sheet. Furthermore, a polyethylene liquid-impermeable back sheet having a size of 12 cm × 32 cm was bonded to a surface on the side opposite to the liquid-permeable top sheet, and thereby an absorbent article for testing was obtained.

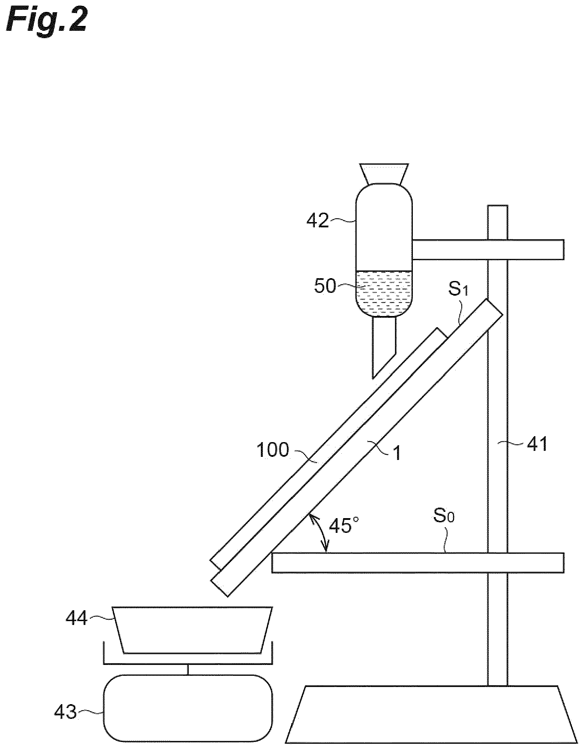

Fig. 2 is a schematic view showing a method for evaluating leakage properties of an absorbent article. A support plate 1 (herein referred to as an acrylic resin plate, and hereinafter also referred to as an inclined plane $S_1$) with a length of 45 cm and having a flat main surface was fixed by a stand 41 in a state of being inclined at 45 ± 2 degrees with respect to a horizontal plane $S_0$. An absorbent article 100 for testing was bonded onto the inclined plane $S_1$ of the fixed support plate 1 in a room at a temperature of 25 ± 2°C such that a longitudinal direction of the absorbent article 100 was along a longitudinal direction of the support plate 1. Next, a test solution 50 (artificial urine) adjusted to 25 ± 1°C was added dropwise from a dropping funnel 42 vertically disposed above the absorbent article toward a position 8 cm, in an upper direction, from the center of an absorbent in the absorbent article 100. 80 mL of the test solution was added dropwise at a time at a speed of 8 mL/sec. A distance between a tip end of the dropping funnel 42 and the absorbent article was 10 ± 1 mm. The test solution was repeatedly added under the same conditions at intervals of 10 minutes from the start of the first addition of the test solution. The test solution was added until leakage was observed.

In a case where the test solution that was not absorbed by the absorbent article 100 leaked out from a lower part of the support plate 1, the leaked test solution was recovered in a metal tray 44 disposed below the support plate 1. A weight (g) of the recovered test solution was measured by a balance 43, and this value was recorded as an amount of leakage. The amount of leakage was subtracted from a total amount of the test solution added to calculate an amount of absorption until the leakage occurred. It is judged that as this numerical value becomes large, liquid leakage is unlikely to occur when the absorbent article is worn. The results are shown in Table 2.

[Table 1]

| | Water retention capacity (g/g) | Moisture content index | | | | Surface drying index (value after 1.5 minutes/ value after 1 minute) × 100 |
|---|---|---|---|---|---|---|
| | | Value after 1 minute | Value after 1.5 minutes | Value after 2 minutes | Value after 3 minutes | |
| Example 1 | 41 | 45 | 10 | 2 | 0 | 22 |
| Example 2 | 41 | 60 | 24 | 9 | 0 | 40 |
| Example 3 | 35 | 23 | 8 | 2 | 0 | 35 |
| Example 4 | 31 | 32 | 4 | 0 | 0 | 12 |
| Comparative Example 1 | 28 | 101 | 13 | 3 | 0 | 13 |
| Comparative Example 2 | 51 | 113 | 78 | 27 | 4 | 69 |
| Comparative Example 3 | 36 | 117 | 116 | 111 | 61 | 99 |

[Table 2]

| | Amount of leakage [g] | | | | | | Amount of absorption until leakage occurs [g] |
|---|---|---|---|---|---|---|---|
| | 1st [g] | 2nd [g] | 3rd [g] | 4th [g] | 5th [g] | 6th [g] | |
| Example 1 | 0 | 0 | 0 | 0 | 26 | - | 374 |
| Example 2 | 0 | 0 | 0 | 0 | 0 | 10 | 470 |
| Example 3 | 0 | 0 | 0 | 0 | 7 | - | 393 |
| Example 4 | 0 | 0 | 0 | 0 | 3 | - | 397 |
| Comparative Example 1 | 0 | 0 | 0 | 21 | 12 | - | 299 |
| Comparative Example 2 | 0 | 0 | 0 | 8 | 35 | - | 312 |
| Comparative Example 3 | 0 | 0 | 5 | - | - | - | 235 |

In the absorbent articles containing the water-absorbent resin particles obtained in the examples, leakage did not occur until the fourth time in the gradient absorption test, and particularly in the absorbent article containing water-absorbent resin particles obtained in Example 2, leakage did not occur until the fifth time. On the other hand, in the absorbent articles containing the water-absorbent resin particles obtained in the comparative examples, leakage occurred at the third time or the fourth time. It was confirmed that occurrence of leakage was sufficiently inhibited in the absorbent articles containing the water-absorbent resin particles obtained in the examples.

**Reference Signs List**

1 support plate, 10 absorbent, 10a water-absorbent resin particle, 10b fiber layer, 20a, 20b...core wrap, 30 liquid-permeable top sheet, 40 liquid-impermeable back sheet, 41 stand, 42 dropping funnel, 43 balance, 44 metal tray, 50 test solution, 100 absorbent article, $S_0$ horizontal plane, $S_1$ inclined plane

**Claims**

1. An absorbent article comprising:

   a laminate that comprises an absorbent, and a core wrap sandwiching the absorbent from upper and lower sides of the absorbent, wherein the absorbent comprises water-absorbent resin particles and a fibrous material;
   a liquid-permeable sheet disposed on an upper surface of the laminate; and
   a liquid-impermeable sheet disposed on a surface on a side opposite to the liquid-permeable sheet of the laminate,
   wherein the water-absorbent resin particles comprise:

   a crosslinked polymer having a monomer unit derived from an ethylenically unsaturated monomer including at least one compound selected from the group consisting of (meth)acrylic acid and a salt thereof, and inorganic particles,

   a proportion of (meth)acrylic acid and a salt thereof is 70 to 100 mol% with respect to a total amount of monomer units in the crosslinked polymer, and
   the water-absorbent resin particles satisfy the following requirements (a) and (b),

   (a) a surface drying index of the absorbent article represented by the following formula is 45 or lower, and
   (b) a water retention capacity of the water-absorbent resin particles for a physiological saline solution is 30 g/g or more,

$$\text{surface drying index} = (\text{moisture content index 1.5 minutes after}$$

$$\text{liquid injection/moisture content index 1 minute after liquid injection}) \times$$

$$100,$$

   a moisture content index measurement method: 160 mL of a 0.9% by mass physiological saline solution (25°C) is injected into a center of the absorbent article using a liquid injection cylinder having an opening with an inner diameter of 3 cm, where a timing of the injection is defined as 0 minutes; and a measurement probe (49 mm$^2$) is pressed on a center portion of the liquid injected site on the absorbent article, and a moisture content index at a surface of the center portion on the absorbent article is measured after a lapse of a predetermined time from the injection by an electrostatic capacitance method.

2. The absorbent article according to claim 1, wherein a moisture content index 3 minutes after the liquid injection is 0.

3. The absorbent article according to claim 1 or 2, wherein a moisture content index 2 minutes after the liquid injection is 2 or higher.

4. The absorbent article according to any one of claims 1 to 3, wherein a moisture content index 1 minute after the liquid injection is 20 or higher.

5. The absorbent article according to any one of claims 1 to 4, wherein a content of the water-absorbent resin particles in the absorbent is 100 to 1,000 g/m$^2$.

6. The absorbent article according to any one of claims 1 to 5, wherein a mass proportion of the water-absorbent resin particles in the absorbent is 10% to 80% with respect to a total amount of the water-absorbent resin particles and the fibrous material.

7. The absorbent article according to any one of claims 1 to 6, wherein a weight per unit area of the liquid-permeable sheet is 5 to 200 g/m$^2$.

8. The absorbent article according to any one of claims 1 to 7, wherein the fibrous material is at least one selected from the group consisting of cellulose-based fibers, synthetic fibers, and a mixture thereof.

9. The absorbent article according to any one of claims 1 to 7, wherein the fibrous material is at least one selected from the group consisting of finely pulverized wood pulp, cotton, cotton linters, rayon, cellulose acetates, polyamides, polyesters, and polyolefins.

10. The absorbent article according to any one of claims 1 to 9, wherein the core wrap is at least one selected from the group consisting of non-woven fabrics, woven fabrics, tissues, synthetic resin films having liquid permeation holes, and net shaped sheets having a mesh.

11. The absorbent article according to any one of claims 1 to 10, wherein the liquid-permeable sheet is at least one selected from the group consisting of thermal bonded non-woven fabric, air through non-woven fabric, spunbond non-woven fabric, and spunbond/melt-blown/spunbond non-woven fabric.

Fig.1

100

30

20a

10b ⎫
10a ⎬ 10

20b

40

# *Fig.2*

# *Fig.3*

## INTERNATIONAL SEARCH REPORT

| | International application No. |
| --- | --- |
| | PCT/JP2019/048795 |

**A. CLASSIFICATION OF SUBJECT MATTER**
A61F 13/53(2006.01)i; C08F 20/06(2006.01)i; C08J 3/12(2006.01)i
FI: A61F13/53 300; C08F20/06; C08J3/12 Z GEY

According to International Patent Classification (IPC) or to both national classification and IPC

**B. FIELDS SEARCHED**

Minimum documentation searched (classification system followed by classification symbols)
A61F13/53; C08F20/06; C08J3/12

Documentation searched other than minimum documentation to the extent that such documents are included in the fields searched
| | |
| --- | --- |
| Published examined utility model applications of Japan | 1922–1996 |
| Published unexamined utility model applications of Japan | 1971–2020 |
| Registered utility model specifications of Japan | 1996–2020 |
| Published registered utility model applications of Japan | 1994–2020 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| P, X<br>P, Y | WO 2019/142872 A1 (SUMITOMO SEIKA CHEMICALS CO., LTD.) 25.07.2019 (2019-07-25) paragraphs [0056], [0076]-[0077], [0081], example 1 (paragraphs [0090]-[0093]), [0131], [0137] | 1-4, 6-11<br>5 |
| Y | JP 2018-166887 A (LIVEDO CORP.) 01.11.2018 (2018-11-01) paragraphs [0072], [0106] | 5 |
| A | JP 2003-088552 A (SUMITOMO SEIKA CHEMICALS CO., LTD.) 25.03.2003 (2003-03-25) production examples 1-3 (paragraphs [0089]-[0093]) | 1-11 |
| A | WO 2018/181565 A1 (SUMITOMO SEIKA CHEMICALS CO., LTD.) 04.10.2018 (2018-10-04) example 2 (paragraphs [0097]-[0101]) | 1-11 |

| ☐ Further documents are listed in the continuation of Box C. | ☒ See patent family annex. |
| --- | --- |

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 19 February 2020 (19.02.2020) | 03 March 2020 (03.03.2020) |

| Name and mailing address of the ISA/<br>Japan Patent Office<br>3-4-3, Kasumigaseki, Chiyoda-ku,<br>Tokyo 100-8915, Japan | Authorized officer<br><br>Telephone No. |
| --- | --- |

Form PCT/ISA/210 (second sheet) (January 2015)

INTERNATIONAL SEARCH REPORT

Information on patent family members

International application No.

PCT/JP2019/048795

| Patent Documents referred in the Report | Publication Date | Patent Family | Publication Date |
|---|---|---|---|
| WO 2019/142872 A1 | 25 Jul. 2019 | (Family: none) | |
| JP 2018-166887 A | 01 Nov. 2018 | WO 2018/179995 A1<br>TW 201836653 A<br>CN 110461291 A | |
| JP 2003-088552 A | 25 Mar. 2003 | (Family: none) | |
| WO 2018/181565 A1 | 04 Oct. 2018 | CN 110446726 A<br>KR 10-2019-0127698 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2007069161 A
- JP 2006167480 A
- JP 2013063254 A
- WO 2011086843 A


**Non-patent literature cited in the description**

- *Pulp and Paper Test Method No. 68,* 2000